# EUROPEAN PATENT APPLICATION

(11) **EP 4 282 364 A1**
(43) Date of publication of application: **29.11.2023**
(21) Application number: 23175315.3
(22) Date of filing: 25.05.2023
(51) Int. Cl.: A61B 18/14, A61B 18/12, A61B 18/00, A61B 17/00

(54) **TRANSSEPTAL TISSUE PUNCTURE APPARATUSES, SYSTEMS, AND METHODS**

(30) Priority: 26.05.2022 US 202217825073
(71) Applicant: BIOSENSE WEBSTER (ISRAEL) LTD., 2066717 Yokneam (IL)
(72) Inventor: GOVARI, Assaf, 2066717 Yokneam (IL); ALTMANN, Andres Claudio, 2066717 Yokneam (IL); MARZIANO, Lilah, 2066717 Yokneam (IL); BEECKLER, Christopher Thomas, Irvine, 92618 (US)
(74) Representative: Carpmaels & Ransford LLP

(57) **Abstract**

A tissue puncture system includes a guidewire and a handle. The guidewire is configured to be inserted into an organ of a patient and to puncture tissue of the organ by conducting an electrical signal from a power source and applying to the tissue electrical current induced between a distal surface of the guidewire and the tissue. The handle is configured to: (i) electrically connect between the power source and a proximal surface of the guidewire, and (ii) move the distal surface to the tissue.

## Description

### FIELD OF THE DISCLOSURE

The present disclosure relates generally to medical devices, and particularly to methods and systems for forming in tissue a transseptal hole, including in some examples by the application of pulsed high-voltage electrical signals using a guidewire.

### BACKGROUND OF THE DISCLOSURE

Various techniques for forming a hole in body tissue have been published.

For example, U.S. Patent 6,565,562 describes a guidewire including a proximal end and a distal end, a distal end including an electrically conductive exposed tip for creating a perforation when RF current is applied and for preventing the creation of a perforation when RF current is not applied.

The present disclosure will be more fully understood from the following detailed description of the examples thereof, taken together with the drawings in which:

### BRIEF DESCRIPTION OF THE DRAWINGS

Fig. 1 is a schematic, pictorial illustration of a catheter-based tracking and ablation system, in accordance with an example of the present disclosure;
Fig. 2 is a schematic, sectional view of a handle mounted upon a guidewire of Fig. 1, in accordance with an example of the present disclosure;
Fig. 3 is a flow chart that schematically illustrate a method for forming a transseptal hole in a heart of a patient, in accordance with an example of the present disclosure; and
Fig. 4 is a flow chart that schematically illustrate a method of using a transseptal puncture system of Fig. 2, in accordance with an example of the present disclosure.

### DETAILED DESCRIPTION OF EXAMPLES

### OVERVIEW

Some medical procedures require tissue puncturing. For example, in tissue ablation of the left atrium of a heart, a catheter is inserted into the right atrium and a small surgical passage is formed in the atrial septum through which the catheter can be directly fed into the left atrium. In principle, it is possible to cut a hole in the septum using a sharp distal end of the catheter (or of another catheter), but such cutting may complicate the procedure and may cause safety issues. For example, (i) in case the position of the hole is inaccurate (e.g., formed at the tissue weakest point rather than at the required point), a physician may have to navigate the catheter in a longer than planned path, and (ii) a larger than planned hole may risk the safety of the patient heart.

Examples of the present disclosure that are described hereafter provide methods and system for improving tissue puncturing using a mounting an attachable handle to a guidewire where the handle creates an electrical connection to an electrical signal generator in order to allow the tip of the guidewire to apply electrical current to a predefined position on the surface of tissue intended to be perforated.

In some examples, a system for puncturing tissue in a patient heart comprises: (i) an electrical signal generator configured to apply electrical pulses having a voltage of at least about 1000 V, in the present example, between about 1200 V and 2000 V, (ii) a catheter which is described in detail below and comprising a guidewire connected (e.g., via a cable) to the electrical signal generator, and (iii) a patch electrode (also referred to herein as an indifferent electrode) attached to the patient skin (e.g., on the patient torso) for perforating the tissue puncturing using electrical current induced by applying a unipolar pulsed field ablation signal, as referred to herein as an irreversible electroporation (IRE) signal and/or an electric field, between the guidewire and the indifferent electrode.

In some examples, the catheter comprises the guidewire, which is configured to be inserted into an organ (e.g., the heart) of the patient and to puncture tissue of the heart by conducting the unipolar IRE signal from the electrical signal generator and applying to the tissue electrical current induced between a distal surface of the guidewire and the tissue.

In some examples, the catheter comprises a handle, which is configured to: (i) electrically connect between the electrical signal generator and a proximal surface of the guidewire, and (ii) move the distal surface of the guidewire to the tissue intended to be perforated.

In some examples, the guidewire is coated with an electrically insulating layer, which is removed from the distal and proximal surfaces for: (i) electrically connecting to the electrical signal generator, and (ii) applying the electrical current to the tissue.

In some examples, the handle comprises (i) an inner hollow tube, which is extended along an axis (e.g., the longitudinal axis) of the handle and is configured to contain a given section of the guidewire, and to electrically isolate the given section from an outer surface of the handle, and (ii) a fixation assembly, which is configured to fixate the proximal surface of the guidewire to a cable that is electrically coupled between the handle and the electrical signal generator.

In some examples, the fixation assembly comprises: (i) a controllable fixation grip, which is positioned at the proximal end of the inner hollow tube, and is electrically connected to the cable, and (ii) a fixation knob, which is configured to control the controllable fixation grip to grip and to release the proximal surface of the guidewire. In the present example, the knob is rotatable about the longitudinal axis, and is configured to perform: (i) the gripping when rotated in a first direction (e.g., clockwise) for tightening the fixation grip to the proximal surface of the guidewire, and (ii) releasing of the proximal surface from the fixation assembly when rotated in a second opposite direction (e.g., counterclockwise). In the present example, the controllable fixation grip comprises one or more electrically conductive plates, which are configured to be moved relative to a wall of the inner hollow tube for gripping and electrically connecting the proximal surface of the guidewire.

In some examples, the distal surface of the guidewire is inserted into a sheath of the catheter having an atraumatic tip. During a medical procedure the physician moves the guidewire toward the surface of a septum between first and second cavities of the heart (e.g., between the right and left atria). Subsequently, the physician extends the distal surface of the guidewire out of the sheath, and places the distal surface at the position intended to be punctured on the septum surface or in close proximity to the surface of the septum. In response to placing the distal surface on the septum and applying the IRE signal, the guidewire is configured to apply the electrical current and to carry out a transseptal puncture in the septum between the right and left atria. In some examples the guidewire may remain recessed within the dilator of the sheath. The dilator may be placed against the septum and energy delivered through the saline inside the dilator without direct contact by the guidewire to the septum.

The disclosed systems, methods, and apparatus, as compared with known conventional traumatic needles and RF needles or guidewires, enables tissue puncturing using nonthermal pulsed electrical current, which improves accuracy, reduces the amount of tissue tenting, improves the size of the transseptal hole formed in the septum, reduces the time required to puncture the septum, all while enabling a more streamlined procedure with fewer exchanges in examples applied in the context of an IRE ablation procedure.

It is noted that, while specific examples disclosed herein refer specifically to the use of high-voltage pulsed voltage (IRE) signals, the handle apparatuses disclosed herein alternatively may be mounted to available guidewires to enable transseptal puncture by the application of RF signals from an electrical signature generator configured to produce RF signals (sometimes referred to as an RF generator) . It is anticipated that the choice between pulsed high-voltage IRE generator or RF generator or a combination thereof may depend on physician preference and/or the modality chosen for the therapeutic phase of the procedure necessitating transeptal access (e.g., ablation in the left atrium) so that a single generator may be used for both the transseptal puncture and ablation phases of the procedure.

### SYSTEM DESCRIPTION

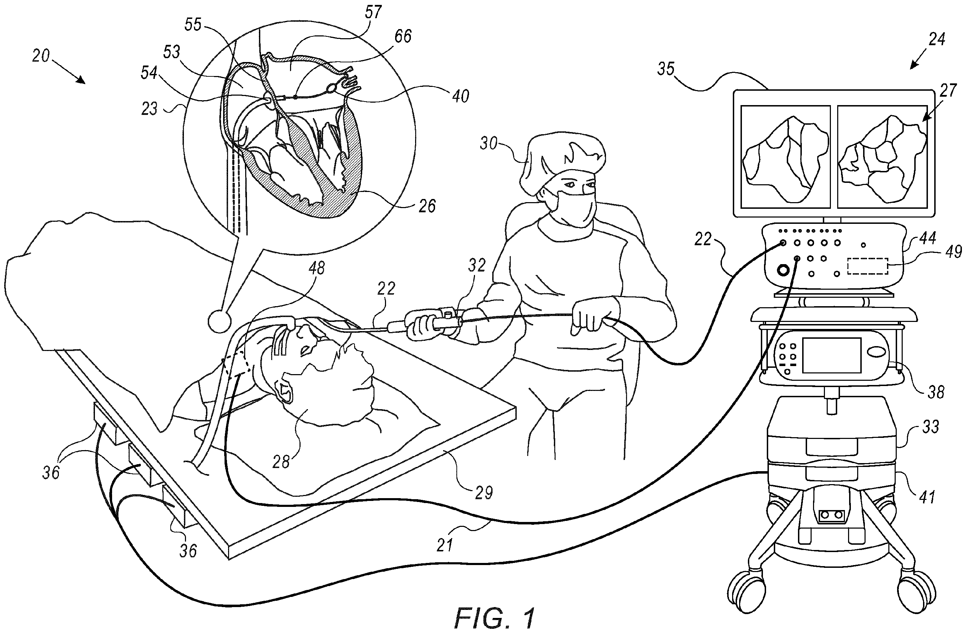

Fig. 1 is a schematic, pictorial illustration of a catheter-based tracking and ablation system 20, in accordance with an example of the present disclosure.

In some examples, system 20 comprises a catheter 22, which is configured to carry out cardiac procedures, and a control console 24. In the example described herein, catheter 22 may be used for any suitable therapeutic and/or diagnostic purposes, such as sensing electro-anatomical signals and/or ablation of tissue in a heart 26. In the context of the present disclosure and in the claims, the term "ablation" refers to a radiofrequency (RF) ablation procedure or to an irreversible electroporation (IRE) procedure, which is different from the RF ablation. IRE may be used, for example, for treating arrhythmia by ablating tissue cells using high voltage applied pulses. Cellular destruction occurs when the transmembrane potential exceeds a threshold, leading to cell death and formation of a lesion. In IRE-based ablation procedures, high-voltage unipolar or bipolar electrical pulses are applied to one or more electrodes in contact with tissue to be ablated, so as to form a lesion in the target location in heart 26, and thereby to treat arrhythmia in the heart. An example of an IRE procedure is described, for example, in U.S. Patent application 16/940,767, published as US2022/0031385, which is incorporated herein in its entirety. The structure and functionality of catheter 22 are described in detail hereinafter.

In some examples, console 24 comprises a processor 33, typically a general-purpose computer, with suitable front end and interface circuits for receiving signals via catheter 22 and for controlling the other components of system 20 described herein. Console 24 further comprises a user display 35, which is configured to receive from processor 33 graphical and/or textual display items, such as a map 27 of heart 26, and to display map 27.

In some examples, map 27 may comprise any suitable type of three-dimensional (3D) anatomical map produced using any suitable technique. For example, the anatomical map may be produced using an anatomical image produced by using a suitable medical imaging system, or using a fast anatomical mapping (FAM) technique available in the CARTO^{™} system, produced by Biosense Webster Inc. (Irvine, Calif.), or using any other suitable technique, or using any suitable combination of the above.

In some examples, console 24 comprises a recording unit 38, which is configured to record in case of failure in the CARTO^{™} and/or a failure to pace in certain electrodes. Console 24 comprises a patient interface unit (PIU) 44, which is configured to produce a signal indicative of the location and electrocardiogram (ECG) signals that are acquired and processed and to exchange signals between console 24 and multiple entities (e.g., catheter 22) of system 20.

Reference is now made to an inset 23. In some examples, prior to performing an ablation procedure, a physician 30 inserts one or more catheters through the vasculature system of a patient 28 lying on a table 29, so as to perform electro-anatomical (EA) mapping of tissue in question of heart 26. Based on the EA mapping, physician 30 plans the RF ablation or IRE procedure.

In some examples, catheter 22 comprises a guidewire 66 and a distal-end assembly 40, such as a balloon catheter having multiple ablation electrodes disposed on the surface of the balloon. In the present example, physician 30 intends to perform an IRE procedure at an intended location on the surface of a left atrium 57 of heart 26. Note that for clinical considerations, the procedure requires: (i) insertion of guidewire 66, through the vasculature system of patient 28, into a right atrium 53 of heart 26, (ii) puncturing a hole 54 (also referred to herein as a transseptal hole) in a septum 55 between right atrium 53 and left atrium 57, (iii) threading guidewire 66 through hole 54 into the intended location in left atrium 57, (iv) moving the balloon along guidewire 66 into the intended location in left atrium 57, and (v) performing the IRE procedure by placing the balloon in contact with the intended tissue and applying the IRE signal (s) to the tissue.

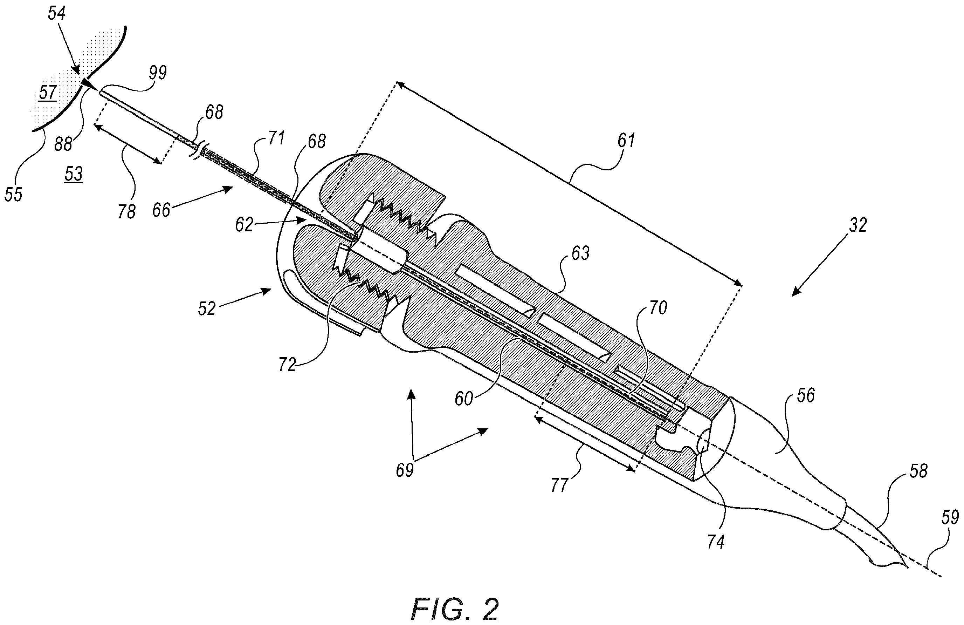

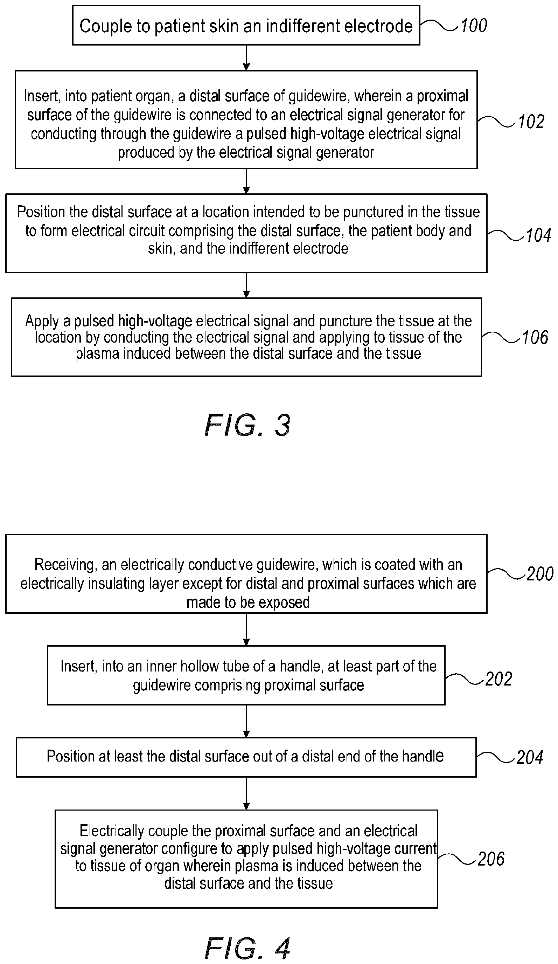

In some examples, the puncturing of hole 54 may be carried out using guidewire 66 or any other guidewire of the same catheter or of another catheter inserted into right atrium 53. In the present example, guidewire 66 comprises any suitable of-the-shelf guidewire, such as a guidewire selected from the Amplatz family of guidewires produced by Boston Scientific Corporation (Marlborough, Massachusetts). For example, Amplatz Super Stiff^{™} Guidewire may be used in the novel manner in conjunction with the systems, apparatus, and methodologies described and claimed herein for puncturing hole 54 in septum 55. The structure and functionality of guidewire 66, and the puncturing process are described in detail in Figs. 2 and 3 below. In other examples, guidewire 66 may comprise any other suitable type of guidewire.

Reference is now made back to the general view of Fig. 1. In some examples, the proximal end of catheter 22 is connected, *inter alia,* to interface circuits (not shown) of PIU 44, for transferring electrocardiogram (ECG) signals sensed by sensing electrodes (not shown) from tissue of heart 26, and position signals (described below) to processor 33 for performing the EA mapping.

In some examples, PIU 44 is connected to a power source, such as an electrical signal generator 49, which is packaged within the housing of PIU 44. In alternative examples, the electrical signal generator may be external to the housing of PIU 44 and electrically connected to PIU 44 using a suitable cable.

In some examples, electrical signal generator 49 is configured to apply unipolar pulsed electrical signals having a voltage of at least 1000 V, in the present example, between about 1200 V and 2000 V. In some examples, system 20 comprises an indifferent electrode 48, also referred to herein as a patch electrode, which is attached to the skin of patient 28 (e.g., on the backside of patient torso) and is electrically connected to PIU 44 via a cable 21. Conveniently, suitable electrical signal generators may be the same used to perform IRE ablation procedures. This obviates the need for a separate electrical generator for each of the transseptal access and ablation phases of a therapeutic procedure. One of many suitable electrical signal generators is disclosed by U.S. Patent Publication No. US20210161592A1, entitled Pulse Generator for Irreversible Electroporation, which is assigned to Biosense Webster Israel Ltd. Further, while unipolar pulsed signals are described in this example, other embodiments may utilize bipolar pulses, particularly where for example e.g., where a guidewire or needle may be outfitted with one or more distal electrode pairs.

In some examples, system 20 is configured to puncture the tissue of septum 55 using electrical induced by applying one or more unipolar pulsed irreversible electroporation (IRE) signals, having about 1200 V or 1800 V or 2000 V (or any other high voltage), between guidewire 66 and indifferent electrode 48. The puncturing components and process are described in more detail in Fig. 2 below. As used herein, a high voltage is at least about 1000 V.

In the context of the present disclosure and in the claims, the terms "about" or "approximately" for any numerical values or ranges indicate a suitable dimensional tolerance that allows the part or collection of components to function for its intended purpose as described herein.

In some examples, after forming hole 54 and inserting distal-end assembly 40 into a target location in left atrium 57, physician 30 may place the ablation electrodes of the balloon in contact with the target location, and apply electrical signals, such as IRE signals or RF ablation signals to the target location.

In some examples, system 20 comprises an attachable handle 32 configured to be mounted to a guidewire and create an electrical connection to an electrical signal generator so that the guidewire may be used by physician 30 to: (i) navigate guidewire 66 into right atrium 53, (ii) produce hole 54 in septum 55, (iii) navigates guidewire 66 to the target location for performing the IRE procedure in left atrium 57, and (iv) moves distal-end assembly 40 for placing the balloon in contact with the target location on the surface of left atrium 57 for applying the one or more IRE signals to the target location. The features of handle 32 are described in detail in Fig. 2 below. In some examples, the handle may be configured to be capable being mounted by an end user (e.g., a medical professional) to a guidewire (including but not limited to commonly available guidewires, as described herein) to form an electrical connection between a distal surface of the guidewire and an electrical signal generator.

In some examples, the position of distal-end assembly 40 in the vasculature and heart 26 of patient 28 is measured using a position sensor (not shown) of a magnetic position tracking system. In the present example, console 24 comprises a driver circuit 41, which is configured to drive magnetic field generators 36 placed at known positions external to patient 28 lying on table 29, e.g., below the patient's torso. The position sensor is coupled to the distal end, and is configured to generate position signals in response to sensed external magnetic fields from field generators 36. The position signals are indicative of the position the distal end of catheter 22 in the coordinate system of the position tracking system.

This method of position sensing is implemented in various medical applications, for example, in the CARTO^{™} system, produced by Biosense Webster Inc. (Irvine, Calif.) and is described in detail in U.S. Patents 5,391,199, 6,690,963, 6,484,118, 6,239,724, 6,618,612 and 6,332,089, in PCT Patent Publication WO 96/05768, and in U.S. Patent Application Publications 2002/0065455 A1, 2003/0120150 A1 and 2004/0068178 A1.

In some examples, the coordinate system of the position tracking system is registered with the coordinate systems of system 20 and map 27, so that processor 33 is configured to display, the position of distal-end assembly 40, over the anatomical or EA map (e.g., map 27).

In some examples, processor 33, typically comprises a general-purpose computer, which is programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

This particular configuration of system 20 is shown by way of example, in order to illustrate certain problems that are addressed by examples of the present disclosure and to demonstrate the application of these examples in enhancing the performance of such a system. Examples of the present disclosure, however, are by no means limited to this specific sort of example system, and the principles described herein may similarly be applied to other sorts of medical systems.

### MOUNTING THE HANDLE AND PERFORATING HEART SEPTUM USING ELECTRICAL CURRENT INDUCED BY APPLYING IRE SIGNAL VIA A GUIDEWIRE

Fig. 2 is a schematic, sectional view of handle 32 and guidewire 66 used for perforating hole 54 in septum 55, in accordance with an example of the present disclosure.

In the context of the present disclosure and in the claims, an assembly comprising at least handle 32, guidewire 66 and electrical signal generator 49 is also referred to herein as a tissue puncturing system or a transseptal puncturing system.

In some examples, handle 32 comprises an inner hollow tube 60, which is extended along an axis 59 (e.g., the longitudinal axis) of handle 32 and is configured to contain a given section 61 of guidewire 66, and to electrically isolate given section 61 from an outer surface 63 of handle 32. In the present example, guidewire 66 is inserted into inner hollow tube 60 through an opening 62 in handle 32.

In some examples, handle 32 comprises a fixation assembly 69, which is configured to fixate a proximal surface 77 of guidewire 66 to an electrical cable, referred to herein as a cable 58, which is comprised in a braid of catheter 22 (and therefore not shown in Fig. 1 above) and is electrically coupled between handle 32 and electrical signal generator 49 (shown in Fig. 1 above). Advantageously, in this example, the disclosed fixation assembly allows handle 32 to be removably attachable to a conventional guidewire 66 to allow the guidewire to become electrically connected to the electrical signal generator 49 for the purpose of electrifying the conductive distal tip 99 for transseptal puncture. The electrical signal generator to which the handle 32 connects the guidewire 66 may be the same generator 49 to provide electrical signals to a therapeutic catheter during the therapeutic phase of the procedure, e.g., IRE or RF ablation, whichever modality may be chosen by the physician. Typically, guidewire 66 is coated with an electrically insulating film 68, such as but not limited to polytetrafluoroethylene (PTFE), polyurethane, polyether block amide (PEBA), or any other suitable type of coating film that is both biocompatible and electrically insulating.

In some examples, electrically insulating film 68 is removed from the distal and proximal surfaces of guidewire 66. More specifically, proximal surface 77 and distal surface 78 of guidewire 66 are electrically conductive and, after removing electrically insulating film 68, their surface is exposed so that proximal surface 77 is electrically coupled to cable 58 using fixation assembly 69, as will be described herein. Cable 58 is configured to conduct IRE pulses of about 1200V or 1800 V or 2000 V (or any other suitable to induce irreversible electroporation of the target tissue, e.g., at least 1000V) between electrical signal generator 49 and handle 32, and therefore, handle 32 comprises an electrical isolation cover 56 to protect physician 30 from being electrified by the high voltage IRE pulses. Handle 32 comprises an opening 74 through which cable 58 connects with proximal surface 77 of guidewire 66. It is noted that in some examples, the supplied guidewire 66 may not have insulating film at the distal and proximal surfaces and thus the removing step may be omitted. And, in yet other examples, where the insulating film is present, the conductive plates of the fixation assembly may comprise electrically conductive sharps. In such examples, the act of attaching the handle, such as by screwing the handle into place using the screw assembly 72, causes the sharps (not shown) to penetrate the insulating film 68 and cause the guidewire to become connected to cable 58 and ultimately to the electrical signal generator 49.

In some examples, fixation assembly 69 comprises: (i) a controllable fixation grip 70, which is positioned at a proximal end of inner hollow tube 60, and is electrically connected to cable 58, and (ii) a fixation knob 52, which is configured to control fixation grip 70 to grip and to release proximal surface 77 of guidewire 66.

In some examples, fixation grip 70 comprises one or more electrically conductive plates, which are electrically connected to cable 58 and are configured to be moved relative to the wall of inner hollow tube 60. In some examples, fixation knob 52 comprises a screw assembly 72, which is configured to move the plates relative to axis 59 and the walls of inner hollow tube 60.

In some examples, in response to rotating fixation knob 52 in a first direction (e.g., clockwise) the plates of fixation grip 70 are moved toward and are coupled with (i.e., gripping) proximal surface 77, and thereby, electrically connecting between cable 58 and proximal surface 77. In response to rotating fixation knob 52 in a second opposite direction (e.g., counterclockwise) the plates of fixation grip 70 are moved away from and are decoupled from proximal surface 77, and thereby, electrically disconnecting between cable 58 and proximal surface 77.

In some examples, guidewire 66 is inserted into a sheath 71 having an atraumatic distal end (not shown) configured to be moved in the vasculature of patient 28 without cutting tissue thereof. As described in Fig. 1 above, physician 30 moves distal surface78 within sheath 71 toward the location intended to be perforated in septum 55, and subsequently, extends distal surface 78 and applies to the intended location electrical current induced by applying the unipolar IRE signal between a distal tip 99 of distal surface 78 of guidewire 66 and indifferent electrode 48 (shown in Fig. 1 above). In some cases, the application of the IRE signal may produce plasma 88 at the vicinity of the induced current. The distal tip 99 of known guidewires are generally electrically thus may serving as an electrode for the application of pulsed high-voltage, when configured in the manner described herein. Accordingly, when configured as descried herein, this may obviate the additional exchange step of inserting a transseptal puncture device (whether a conventional sharp or RF needle guidewire), as typically required.

In some examples, based on position signals from one or more position sensors (not shown) coupled, e.g., to guidewire 66, processor 33 is configured to display the position of distal surface 78 and/or distal tip 99 on map 27. Alternatively, guidewire 66 may be displayed using impedancebased location sensing. In such examples, physician 30 navigates distal surface 78 to the desired location on the surface of septum 55 and electrical current, which is induced in response to the application of the IRE signal to guidewire 66, punctures hole 54 in the tissue at the desired location in septum 55. Note that when inserting distal-end assembly 40 into left atrium 57 every excess movement of catheter 22 may increase the size of hole 54 in septum 55. Therefore, it is important to form a transseptal hole, such as hole 54, at an accurate position to reduce excess movement of catheter 22 while moving the balloon to ablate the target tissue in left atrium 57. Moreover, the unipolar pulsed high-voltage electrical current that is discharged enables puncturing septum 55 in less than about one second, which is faster than cutting septum 55 using a sharp edge of a needle or perforating septum 55 by applying an RF signal by RF needle or guidewire. Note that a shorter puncturing duration may improve the quality of hole 54 and reduce the damage that may be caused to heart 26 during the puncturing process. As noted previously, because disclosed example systems and methods may utilize the same electrical signal generator and guidewire used to perform ablation procedures, the fewer exchanges may be necessary as compared with other transseptal puncture procedures that require the separate step of inserting a conventional sharp or RF needle.

This particular configuration of system 20, handle 32 and guidewire 66, is shown by way of example, in order to illustrate certain problems that are addressed by examples of the present invention and to demonstrate the application of these examples in enhancing the performance of such a system configured for tissue puncturing and puncturing of the septum in particular. Examples of the present invention, however, are by no means limited to this specific sort of example system or handle, and the principles described herein may similarly be applied to other sorts of handles, manipulators, and medical systems.

Fig. 3 is a flow chart that schematically illustrate a method for puncturing a transseptal hole, such as hole 54 in septum 55 of heart 26, in accordance with examples of the present disclosure.

The method begins at an indifferent electrode coupling step 100, with physician 30, or any other user of system 20, coupling to the skin of patient 28 a patch comprising indifferent electrode 48, as described in Fig. 1 above.

At a guidewire insertion step 102, physician 30 inserts at least distal surface 78 of guidewire 66 of catheter 22, into the vasculature of patient 28 and use handle 32 to navigate surface 78 into right atrium 53 of heart 26. Note that: (i) at least surface 78 is inserted into sheath 71 to prevent damage to any tissue of patient 28 while navigating surface 78 into right atrium 53, and (ii) at least part of guidewire 66 (including proximal surface 77) is inserted into inner hollow tube 60 of handle 32, and proximal surface 77 is electrically connected to electrical signal generator 49 via controllable fixation grip 70 and cable 58, as described in detail in Fig. 2 above.

At guidewire positioning step 104, physician 30 positions distal surface 78 and more particularly distal tip 99 at a location intended to be punctured in the tissue of septum 55, and extends the distal surface 78 to expose distal tip 99 to the tissue so as to form an electrical circuit comprising distal surface 78, the body of patient 28, and indifferent electrode 48.

In some examples, in response to applying one or more IRE signals when distal surface 78 is in close proximity to or placed in contact with tissue of septum 55, guidewire 66 is configured to apply to the tissue, an electrical current induced between distal surface 78 and the tissue, as described in detail in Figs. 1 and 2 above. Plasma 88 may be formed in response to the discharge of electrical current.

At a puncturing step 106 that concludes the method, physician 30 uses processor 33 for controlling electrical signal generator 49 to apply the one or more IRE signals, and punctures hole 54 at the location on the surface of septum 55 by applying to septum 55 electrical current (which is induced by conducting the IRE signals) between distal surface 78 and the tissue of septum 55.

In some examples, after forming hole 54 in septum 55, physician may insert, through hole 54 into left atrium 57, distal-end assembly 40 having the balloon for applying one or more IRE pulsed signals and/or RF ablation signals to a target tissue of left atrium 57. Additionally, or alternatively, physician 30 may insert, through hole 54 into left atrium 57, any other suitable type of catheter for performing in left atrium 57 any other suitable medical procedure.

In alternative examples, the method of Fig. 3 may be used, *mutatis mutandis,* for puncturing any other tissue applied to any other organ of patient 28 or any other patient, so as to perform any suitable type of a medical procedure.

Fig. 4 is a flow chart that schematically illustrate a method for producing a transseptal puncturing system comprising handle 32 and guidewire 66, in accordance with an example of the present disclosure.

The method begins at a coating removal step 200, with receiving guidewire 66 and removing electrically insulating film 68 from proximal and distal surfaces 77 and 78 of guidewire 66. In other examples, surfaces 77 and 78 of guidewire 66 may already be exposed (i.e., not being coated with electrically insulating film 68), so that step 200 may be eliminated from the method.

At a guidewire insertion step 202, at least part of guidewire 66 that comprises proximal surface 77, is inserted into inner hollow tube 60 of handle 32, as shown and described in Fig. 2 above.

At a distal surface positioning step 204, at least surface 78 (and typically additional sections) of guidewire 66 is positioned out of opening 62 at the distal end of handle 32, as described in Fig. 2 above.

At a proximal surface coupling step 206 that concludes the method, surface 77 is electrically coupled to electrical signal generator 49 via the electrically conductive plates of controllable fixation grip 70 and cable 58. In some examples, in response to producing one or more IRE signals by electrical signal generator 49, when physician 30 places the distal surface 78 in close proximity or in contact with any tissue of patient 28, such as the surface of septum 55, electrical current is induced between distal surface 78 and the respective tissue, as described in detail in Figs. 2 and 3 above.

As described above, during the medical procedure physician 30 may insert distal-end assembly 40 over guidewire 66 into left atrium 57. In some examples, the method may comprise additional steps related to the production of distal-end assembly 40 and/or other components related to catheter 22 and other parts of system 20.

Although the examples described herein mainly address puncturing a transseptal hole in patient heart, the methods and systems described herein can also be used in other applications, such as in tissue puncturing applied to any other organ of a patient.

### Example 1:

A transseptal puncture system (22), including:
(a) a guidewire (66), which is configured to be inserted into an organ (26) of a patient and to puncture tissue (55) of the organ (26) by conducting an electrical signal from a power source (49) and applying to the tissue (55) electrical current induced between a distal surface (78) of the guidewire (66) and the tissue (55); and
(b) a handle (32), which is configured to: (i) electrically connect between the power source (49) and a proximal surface (77) of the guidewire (66), and (ii) to move the distal surface (78) to the tissue (55).

### Example 2:

The system according to example 1, wherein at least a section of the guidewire, other than the first and proximal surfaces, is coated with an electrically insulating layer.

### Example 3:

The system according to example 1, wherein the handle includes: (i) an inner hollow tube, which is extended along an axis of the handle and is configured to contain a given section of the guidewire, and to electrically isolate the given section from an outer surface of the handle, and (ii) a fixation assembly, which is configured to fixate the proximal surface to a cable that is electrically coupling between the handle and the power source.

### Example 4:

The system according to example 3, wherein the fixation assembly includes: (i) a controllable fixation grip, which is positioned at a proximal end of the inner hollow tube, and is electrically connected to the cable, and (ii) a fixation knob, which is configured to control the controllable fixation grip to grip and to release the proximal surface of the guidewire.

### Example 5:

The system according to example 4, wherein the controllable fixation grip includes one or more electrically conductive plates, which are electrically connected to the cable and are configured to be moved relative to a wall of the inner hollow tube, and wherein the fixation knob is configured to move the one or more electrically conductive plates in: (i) a first direction for coupling between the one or more electrically conductive plates and the proximal surface, and (ii) a second direction for decoupling between the one or more electrically conductive plates and the proximal surface.

### Example 6:

The system according to examples 1 through 5, wherein the electrical signal includes a radiofrequency (RF) signal having at least 1000 volts, wherein the organ includes a heart and the tissue includes a septum between first and second cavities of the heart, and wherein, in response to placing the distal surface on the septum and applying the electrical signal, the guidewire is configured to carry out a transseptal puncture in the septum between the first and second cavities.

### Example 7:

The system according to example 6, wherein the electrical signal includes a pulsed irreversible electroporation signal.

### Example 8:

The system according to examples 1 through 7, and including an indifferent electrode coupled to a skin of the patient, to form an electrical circuit including the distal surface, a body and the skin of the patient, and the indifferent electrode, and wherein the electrical signal includes a unipolar signal.

### Example 9:

The system according to examples 1 through 8, and including a sheath, which is configured to fit over at least the distal surface at least when inserting the distal surface into the patient.

### Example 10:

The system according to example 6, wherein the electrical signal comprises a bipolar pulsed signal.

### Example 11:

A method, including:
mounting a handle to a guidewire whereby the mounting the handle creates an electrical connection between a distal tip of the guidewire and an electrical signal generator;
inserting, into an organ of a patient, at least a distal surface of a guidewire, and applying to tissue of the organ an electrical current induced between the distal surface and the tissue;
positioning the distal surface at a location intended to be punctured in the tissue; and
puncturing the tissue at the location by conducting the electrical signal and applying the electrical current between the distal surface and the tissue.

### Example 12:

The method according to example 11, wherein the electrical signal includes an electrical signal having a voltage at least 1000 volts, wherein the organ includes a heart, and the tissue includes a septum between first and second cavities of the heart, and wherein puncturing the tissue includes forming a transseptal puncture in the septum between the first and second cavities.

### Example 13:

The method according to example 12, wherein the electrical signal includes a pulsed irreversible electroporation signal.

### Example 14:

The method according to examples 11 through 13, and including coupling, to a skin of the patient, an indifferent electrode to form an electrical circuit including the distal surface, a body and the skin of the patient and the indifferent electrode.

### Example 15:

A method for producing a transseptal puncturing system, the method including:
inserting, into an inner hollow tube of a handle, at least part of an electrically conductive guidewire having: (i) first and proximal surface sections, and (ii) an additional section between the first and proximal surface section, which is coated with an electrically insulating layer;
positioning at least the distal surface section out of a distal end of the handle; and
electrically coupling between the proximal surface section and a power source configured to produce an electrical signal for applying, to tissue of an organ, electrical current induced between the distal surface section and the tissue.

### Example 16:

The method according to example 15, and including removing the electrically insulating layer from the first and proximal surface sections.

### Example 17:

The method according to examples 15 through 16, wherein the handle includes a fixation assembly, which is configured to fixate the proximal surface to a cable that is electrically coupling between the handle and the power source, and the inner hollow tube is extended along an axis of the handle and is configured to contain at least the proximal surface section of the guidewire, and to electrically isolate at least the proximal surface section from an outer surface of the handle.

### Example 18:

The method according to example 17, wherein the fixation assembly includes: (i) a controllable fixation grip, which is positioned at a proximal end of the inner hollow tube, and is electrically connected to the cable, and (ii) a fixation knob, which is configured to control the controllable fixation grip to grip and to release the proximal surface of the guidewire.

### Example 19:

The method according to example 18, wherein the controllable fixation grip includes one or more electrically conductive plates, which are electrically connected to the cable and are configured to be moved relative to a wall of the inner hollow tube, wherein: (i) electrically coupling between the proximal surface section and the power source includes moving the one or more electrically conductive plates in a first direction, and (ii) electrically decoupling between the proximal surface section and the power source includes moving the one or more electrically conductive plates in a second direction.

### Example 20:

The method according to examples 15 through 19, wherein the power source is configured to produce a pulsed irreversible electroporation signal having a voltage of at least 1000 volts.

It will be appreciated that the examples described above are cited by way of example, and that the present disclosure is not limited to what has been particularly shown and described hereinabove. Rather, the scope of the present disclosure includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

## Claims

1. A tissue puncture system (22), comprising:
a guidewire (66), which is configured to be inserted into an organ (26) of a patient and to puncture tissue (55) of the organ (26) by conducting an electrical signal from a power source (49) and applying to the tissue (55) electrical current induced between a distal surface (78) of the guidewire (66) and the tissue (55); and
a handle (32), which is configured to: (i) electrically connect between the power source (49) and a proximal surface (77) of the guidewire (66), and (ii) move the distal surface (78) to the tissue (55).

2. The system according to claim 1, wherein at least a section of the guidewire, other than the first and proximal surfaces, is coated with an electrically insulating layer.

3. The system according to claim 1, wherein the handle comprises: (i) an inner hollow tube, which is extended along an axis of the handle and is configured to contain a given section of the guidewire, and to electrically isolate the given section from an outer surface of the handle, and (ii) a fixation assembly, which is configured to fixate the proximal surface to a cable that is electrically coupling between the handle and the power source.

4. The system according to claim 3, wherein the fixation assembly comprises: (i) a controllable fixation grip, which is positioned at a proximal end of the inner hollow tube, and is electrically connected to the cable, and (ii) a fixation knob, which is configured to control the controllable fixation grip to grip and to release the proximal surface of the guidewire.

5. The system according to claim 4, wherein the controllable fixation grip comprises one or more electrically conductive plates, which are electrically connected to the cable and are configured to be moved relative to a wall of the inner hollow tube, and wherein the fixation knob is configured to move the one or more electrically conductive plates in: (i) a first direction for coupling between the one or more electrically conductive plates and the proximal surface, and (ii) a second direction for decoupling between the one or more electrically conductive plates and the proximal surface.

6. The system according to any preceding claim, wherein the electrical signal comprises a voltage of at least 1000 volts, wherein the organ comprises a heart and the tissue comprises a septum between first and second cavities of the heart, and wherein, in response to placing the distal surface on the septum and applying the electrical signal, the guidewire is configured to carry out a transseptal puncture in the septum between the first and second cavities.

7. The system according to claim 6, wherein the electrical signal comprises a pulsed irreversible electroporation signal.

8. The system according to any preceding claim, and comprising an indifferent electrode coupled to a skin of the patient, to form an electrical circuit comprising the distal surface, a body and the skin of the patient, and the indifferent electrode, and wherein the electrical signal comprises a unipolar signal.

9. The system according to claim 6, wherein the electrical signal comprises a unipolar pulsed signal.

10. The system according to claim 6, wherein the electrical signal comprises a bipolar pulsed signal.

11. A method for producing a transseptal puncturing system, the method comprising:
inserting, into an inner hollow tube of a handle, at least part of an electrically conductive guidewire having: (i) first and proximal surface sections, and (ii) an additional section between the first and proximal surface section, which is coated with an electrically insulating layer;
positioning at least the distal surface section out of a distal end of the handle; and
electrically coupling between the proximal surface section and a power source configured to produce an electrical signal for applying, to tissue of an organ, electrical current induced between the distal surface section and the tissue.

12. The method according to claim 11, and comprising removing the electrically insulating layer from the first and proximal surface sections.

13. The method according to claim 11 or claim 12, wherein the handle comprises a fixation assembly, which is configured to fixate the proximal surface to a cable that is electrically coupling between the handle and the power source, and the inner hollow tube is extended along an axis of the handle and is configured to contain at least the proximal surface section of the guidewire, and to electrically isolate at least the proximal surface section from an outer surface of the handle.

14. The method according to claim 13, wherein the fixation assembly comprises: (i) a controllable fixation grip, which is positioned at a proximal end of the inner hollow tube, and is electrically connected to the cable, and (ii) a fixation knob, which is configured to control the controllable fixation grip to grip and to release the proximal surface of the guidewire.

15. The method according to claim 14, wherein the controllable fixation grip comprises one or more electrically conductive plates, which are electrically connected to the cable and are configured to be moved relative to a wall of the inner hollow tube, wherein: (i) electrically coupling between the proximal surface section and the power source comprises moving the one or more electrically conductive plates in a first direction, and (ii) electrically decoupling between the proximal surface section and the power source comprises moving the one or more electrically conductive plates in a second direction.

16. The method according to any of claims 11 to 15, wherein the power source is configured to produce a pulsed irreversible electroporation signal having a voltage at least 1000 volts.
